# FASCICULE DE BREVET EUROPEEN

(11) **EP 3 988 149 B1**
(45) Date de publication et mention de la délivrance du brevet: **21.02.2024**
(21) Numéro de dépôt: 21189547.9
(22) Date de dépôt: 04.08.2021
(51) Int. Cl.: A61M 16/00, H02H 9/04, H02P 6/00, H02H 7/08, H02H 7/09, F04D 27/00

(54) **SYSTÈME DE CONTRÔLE DE LA MICRO-SOUFFLANTE MOTORISÉE D'UN APPAREIL D'ASSISTANCE RESPIRATOIRE**
STEUERUNGSSYSTEM FÜR DAS MOTORISIERTE MIKROGEBLÄSE EINES GERÄTS ZUR ATMUNGSUNTERSTÜTZUNG
SYSTEM FOR CONTROLLING THE MOTORISED MICRO-FAN OF A RESPIRATORY ASSISTANCE DEVICE

(30) Priorité: 23.10.2020 FR 2010916
(43) Date de publication de la demande: 27.04.2022
(73) Titulaire: EOVE, 64000 Pau (FR)
(72) Inventeur: COTTEAUX, Fabien, 64000 Pau (FR); LADAURADE, Nicolas, 64000 Pau (FR)
(74) Mandataire: Air Liquide

(56) Documents cités:
- EP-A1- 2 827 489
- EP-A1- 3 242 330
- EP-A1- 3 300 121
- WO-A1-2015/000025
- DE-T5-112014 006 358
- US-A1- 2018 278 183

## Description

L'invention porte sur un système de contrôle du fonctionnement de la micro-soufflante motorisée d'un appareil d'assistance respiratoire, c'est-à-dire un ventilateur médical, servant à fournir un gaz respiratoire, tel un flux d'air enrichi ou non en oxygène, à un patient pour traiter une ou des pathologies respiratoires chez ce patient, en particulier des accélérations et freinages de la micro-soufflante.

Afin d'assister certains patients dans leur fonction respiratoire, on utilise des appareils d'assistance respiratoire, encore appelés « ventilateurs médicaux », délivrant un gaz respiratoire, tel de l'air ou de l'air enrichi en oxygène, à débit non nul et/ou à une pression supérieure à la pression atmosphérique (> 1 atm).

Ces appareils d'assistance respiratoire mettent en oeuvre une micro-soufflante motorisée, aussi appelée « soufflante », « compresseur » ou « turbine », servant à aspirer de l'air ambiant et à le délivrer à une pression donnée aux patients.

L'aspiration de l'air par la micro-soufflante, pendant le fonctionnement de son moteur électrique, se fait grâce à une ou plusieurs roues à ailettes agencées sur un arbre ou axe rotatif entraîné en rotation par un moteur électrique, la (ou les) roue(s) à ailettes étant mobile(s) en rotation dans le compartiment interne d'une volute.

L'air peut être enrichi en oxygène, c'est-à-dire additionné d'oxygène supplémentaire, notamment lorsque le patient doit recevoir, dans le cadre de son traitement, une proportion d'oxygène supérieure à 21 % en volume. L'oxygène peut être mélangé à l'air en amont ou en aval du compartiment interne de la volute.

Des exemples de telles technologies sont donnés par EP-A-2165078, EP-A-2102504, WO-A-2012/139681, US-A-2008/304986 et WO-A-2013/020167. Un autre exemple d'un ventilateur médical connu est divulgué dans le document WO 2015/000025 A1.

Pendant le fonctionnement du ventilateur médical, le moteur électrique de la micro-soufflante est commandé par des moyens de contrôle du ventilateur médical et subit cycliquement des phases d'accélération et de décélération, i.e. de freinage ou ralentissement, successives se calquant sur les phases inspiratoires et expiratoires du patient, respectivement.

Or, lors des phases de freinage, c'est-à-dire des décélérations ou des ralentissements, du moteur électrique, on constate l'existence de surtensions électriques importantes qui sont générées par le moteur, lors du relâchement du court-circuit de chaque phase moteur en situation de freinage.

Plus précisément, de l'énergie emmagasinée dans les bobines du moteur électrique est subitement relâchée dans le bus d'alimentation reliant la source de courant au moteur, en provoquant une élévation de la tension d'alimentation générale et pouvant engendrer une détérioration des composants situés sur ce bus.

En outre, ces phénomènes sont néfastes pour le ventilateur médical car ils réduisent les performances, notamment des moyens de contrôle du moteur électrique et affectent leur durée de vie.

Il est donc nécessaire de protéger les composants sensibles du ventilateur médical contre les surtensions ou autres pics d'énergie pouvant se produire pendant le fonctionnement du ventilateur, en particulier lors des phases de freinage du moteur électrique de la micro-soufflante, c'est-à-dire lors des décélérations ou ralentissements du moteur.

US-A-2011/162647 tente de résoudre ce problème en récupérant l'énergie de freinage et en la stockant dans la batterie du ventilateur médical. Cette solution est cependant compliquée à mettre en oeuvre car elle nécessite la mise en place d'un circuit de récupération d'énergie spécifique et assez complexe, dont le rendement est discutable.

EP-A-3017345 propose quant à lui d'installer d'un circuit à diode d'absorption transitoire, i.e. avec une ou des diodes d'absorption de tensions transitoires ou TVS (i.e. *Transient Voltage Suppressor*)*,* sur le bus entre alimentation électrique et moteur pour absorber l'énergie générée lors du freinage. Toutefois, un tel circuit bloqueur de transitoires agencé sur le bus n'est pas idéal car il ne permet pas de toujours protéger efficacement les composants, du fait d'un seuil de protection de ce type de diodes assez imprécis.

D'autres documents proposent différentes configurations de circuits, notamment US2018/278183, EP3300121, EP3242330, DE112014006358 et JP2827489. Toutefois, aucun de ces documents ne concernent le domaine des ventilateurs médicaux et encore moins la dissipation des surtensions électriques importantes générées par le moteur de la micro-soufflante d'un tel ventilateur médical, lors des phases de freinage, c'est-à-dire des décélérations ou des ralentissements, du moteur électrique, de manière à protéger les composants sensibles du ventilateur médical contre les surtensions ou autres pics d'énergie.

Au vu de cela, le problème est donc de proposer un appareil d'assistance respiratoire, c'est-à-dire un ventilateur médical, équipé d'un système de contrôle de la micro-soufflante motorisée qui permette un contrôle efficace des accélérations et décélérations (i.e. freinages/ralentissements) de la micro-soufflante et qui soit par ailleurs protégé efficacement contre les surtensions électriques, les pics d'énergie ou analogues, engendrés lors des phases de décélération/freinage du moteur électrique de la micro-soufflante, de manière à éviter ou à limiter la détérioration des composants électroniques sensibles à ces surtensions électriques ou analogues.

L'invention est définie dans la revendication indépendante 1. Les modes de réalisation préférés sont décrits dans les revendications dépendantes.

L'invention porte sur un appareil d'assistance respiratoire, c'est-à-dire un ventilateur médical, pour fournir un gaz respiratoire à un patient (i.e. un être humain) comprenant :
- une micro-soufflante équipée d'un moteur électrique, c'est-à-dire une micro-soufflante motorisée,
- un système de contrôle de moteur pour contrôler le moteur électrique de la micro-soufflante, et
- une alimentation électrique alimentant électriquement le moteur électrique et le système de contrôle via un bus d'alimentation reliant l'alimentation électrique au moteur électrique de la micro-soufflante.

Selon l'invention, le système de contrôle comprend :
- un circuit à ponts de transistors, agencé entre l'alimentation électrique et le moteur électrique, comprenant 3 ponts de transistors agencés en parallèle, chaque pont de transistors reliant le bus au moteur et comprenant un couple de transistors,
- un circuit de pilotage du moteur pour commander au moins les accélérations et les décélérations (i.e. freinages ou ralentissements) du moteur, ledit circuit de pilotage du moteur étant relié électriquement au moteur et aux transistors, et
- une pluralité de diodes de régulation de tension agencées en parallèle des transistors pour limiter au moins une partie des surtensions (i.e. pics de tension ou d'énergie) du moteur lors des décélérations (i.e. freinages ou ralentissements) du moteur.

Dans le cadre de la présente invention, par « diode de régulation de tension », on entend une diode permettant d'avoir un seuil de tension inverse fixe, c'est-à-dire égal à une valeur donnée variant peu ou pas en fonction du courant, typiquement une diode Zener ou analogue, et ce, contrairement à une diode TVS qui a une tension variant de manière importante avec le courant, c'est-à-dire qui est une diode à tension variable.

Selon le mode de réalisation considéré, l'appareil d'assistance respiratoire, c'est-à-dire le ventilateur médical, selon l'invention peut comprendre l'une ou plusieurs des caractéristiques suivantes :
- il comprend en outre, agencés entre l'alimentation électrique et le moteur électrique, un dispositif interrupteur pour contrôler le passage du courant dans le bus d'alimentation en direction du moteur.
- le dispositif interrupteur comprend un transistor MOS.
- le dispositif interrupteur est commandé par le circuit de pilotage.
- il comprend en outre agencée entre l'alimentation électrique et le moteur électrique, au moins une diode de blocage de courant remontant pour bloquer le courant généré par le moteur et circulant dans le bus d'alimentation en direction de l'alimentation électrique, lors des décélérations du moteur, i.e. freinages ou analogues.
- ladite au moins une diode de blocage de courant remontant est au moins une diode Schottky ou analogue.
- il comprend en outre agencée entre l'alimentation électrique et le moteur électrique, typiquement sur le bus, au moins une diode d'écrêtage, i.e. une diode de régulation de tension additionnelle, servant à limiter au moins une partie des surtensions de l'alimentation électrique circulant dans le bus.
- ladite au moins une diode d'écrêtage est au moins une diode Zener ou analogue.
- il comprend en outre au moins un condensateur, agencée entre l'alimentation électrique et le moteur électrique, pour fournir du courant au moteur, lors des accélérations du moteur, et pour limiter au moins une partie des surtensions du moteur lors des décélérations du moteur.
- le dispositif interrupteur et/ou la ou les diodes de blocage de courant remontant sont agencés sur le bus d'alimentation, en particulier sur la branche (+) du bus.
- il comprend par ailleurs un circuit limiteur de courant configuré pour limiter le courant circulant dans le bus vers le moteur, lors de la fermeture du dispositif interrupteur.
- le circuit limiteur de courant comprend un circuit RC à résistance et condensateur, configuré pour fixer une constante de temps de commande du dispositif interrupteur.
- le circuit limiteur de courant est commandé par le circuit de pilotage, de préférence un microcontrôleur du circuit de pilotage.
- le circuit à ponts de transistors comprend un premier, un deuxième et un troisième ponts de transistors et le moteur comprend une première, une deuxième et une troisième phases, les premier, deuxième et troisième ponts de transistors étant respectivement reliés aux première, deuxième et troisième phases du moteur, c'est-à-dire que chaque pont de transistors est relié électriquement à uniquement l'une des 3 phases du moteur et chacune des 3 phases du moteur est reliée électriquement à uniquement l'un de 3 pont de transistors.
- les première, deuxième et troisième phases du moteur sont, respectivement, reliées électriquement aux premier, deuxième et troisième ponts de transistors respectivement entre les deux transistors de chaque couple de transistors et entre les deux diodes de régulation de tension des couples diodes de régulation de tension agencés en parallèle desdits transistors.
- les diodes de régulation de tension sont des diodes Zener ou analogue.
- les diodes de régulation de tension ne sont pas des diodes TVS de suppression des transitoires, notamment pas des diodes de type Transil^{®} ou Transzorb^{®}.
- la micro-soufflante est équipée d'un moteur électrique sans balai (i.e. « brushless » en anglais).
- le circuit de pilotage du moteur comprend un microprocesseur, de préférence il comprend un microcontrôleur.
- le circuit de pilotage du moteur comprend un microprocesseur agencé sur une carte électronique, i.e. carte à circuit imprimé.
- le bus d'alimentation comprend des liaisons électriques de types circuits imprimés, câbles ou fils électriques.
- l'alimentation électrique comprend une ou plusieurs batteries rechargeables et/ou un cordon électrique, un convertisseur AC/DC et une prise de raccordement au secteur (i.e. 110/220 V).
- l'alimentation électrique est de type AC/DC (tension alternative/ tension continue) fournissant une tension de moins de 30 V (tension continue), par exemple de l'ordre de 28 V.
- l'alimentation électrique fournit du courant électrique à tous les composants ou éléments de l'appareil nécessitant du courant électrique pour fonctionner, notamment au moteur de la micro-soufflante, au circuit de pilotage etc...
- la micro-soufflante est commandée par le circuit de pilotage du moteur pour fonctionner selon des cycles alternant des phases de fourniture de gaz et de non-fourniture (i.e. arrêt) de gaz au patient.
- pendant les phases de fourniture de gaz, le moteur de la micro-soufflante subit des accélérations.
- pendant les phases de non-fourniture de gaz, le moteur de la micro-soufflante subit des décélérations, c'est-à-dire des freinages ou des ralentissements.
- le moteur électrique de la micro-soufflante est protégé par un carter de protection externe, de préférence rigide.
- la micro-soufflante comprend une volute délimitant un compartiment interne dans lequel est agencée la roue à ailettes.
- la micro-soufflante est équipée d'un moteur électrique entraînant en rotation un arbre, aussi appelé axe rotatif, portant une (ou des) roue à ailettes.
- la volute comprend une ouverture supérieure servant d'entrée de gaz dans le compartiment interne, ledit gaz étant aspiré par la roue à ailettes, durant son fonctionnement.
- la volute comprend un conduit de sortie de gaz en communication fluidique avec le compartiment interne, pour extraire dudit compartiment interne, un flux gazeux généré par la roue à ailette, lorsque ladite roue à ailette est entraînée en rotation par l'arbre.
- la volute est formée de deux demi-volutes, à savoir une volute inférieure et une volute supérieure fixées l'une à l'autre de manière étanche.
- les deux demi-volutes définissent entre elles le compartiment interne.
- la micro-soufflante délivre de l'air ou un mélange air/oxygène.
- il comprend par ailleurs un caisson, i.e. boitier ou analogue, au sein duquel est agencée la micro-soufflante motorisée.
- le caisson comprend une entrée d'air en communication fluidique avec un orifice d'entrée d'air par laquelle l'air pénètre dans l'appareil.
- selon un mode de réalisation, le caisson comprend une entrée d'oxygène en communication fluidique avec un orifice d'entrée d'oxygène, typiquement un raccord d'alimentation en oxygène, par laquelle de l'oxygène provenant d'une source d'oxygène, telle une bouteille d'oxygène ou une canalisation d'amenée d'oxygène, peut pénétrer dans l'appareil.
- le caisson comprend une sortie de flux gazeux (e.g. air ou air/O₂) en communication fluidique avec le conduit de sortie de gaz de la volute.
- le caisson est formé de polymère ou tout autre matériau.
- selon un mode de réalisation, le caisson est formé de deux parties de caisson, c'est-à-dire typiquement de 2 demi-caissons, fixés l'un à l'autre.
- le caisson est agencé dans la carcasse du ventilateur médical.
- alternativement, la micro-soufflante est agencée directement dans la carcasse du ventilateur médical.
- le moteur est commandé pour tourner à une vitesse pouvant atteindre 50 000 à 70 000 tr/min, typiquement de 30 000 à 40 000 tr/min.
- le moteur électrique comprend un rotor et un stator.
- il comprend une IHM ou interface homme-machine.
- il comprend un écran de visualisation, i.e. d'affichage, d'informations.
- il est de type à pression continue (CPAP), à débit continu, à deux niveaux de pression (BiPAP), ou autre, et/ou d'urgence.

L'invention porte aussi sur une installation de fourniture de gaz respiratoire à un patient comprenant un appareil d'assistance respiratoire selon l'invention, un conduit flexible raccordé fluidiquement audit appareil d'assistance respiratoire et alimenté en gaz par la micro-soufflante dudit appareil d'assistance respiratoire, et une interface respiratoire, par exemple un masque, des canules nasales, une sonde trachéale ou analogue, alimentée en gaz par ledit conduit flexible, typiquement en air ou mélange air/O₂.

Avantageusement, une source d'oxygène, telle une bouteille d'oxygène sous pression, est reliée fluidiquement à l'appareil d'assistance respiratoire selon l'invention afin de lui fournir de l'oxygène permettant d'enrichir de l'air et obtenir un mélange air/O₂.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un appareil d'assistance respiratoire à micro-soufflante motorisée selon l'invention,
Fig. 2 détaille le compartiment à micro-soufflante de l'appareil de Fig. 1,
Fig. 3 est une vue schématique de côté de la micro-soufflante de Fig. 2 et
Fig. 4 schématise un mode de réalisation d'un système de contrôle du moteur de la micro-soufflante selon l'invention.

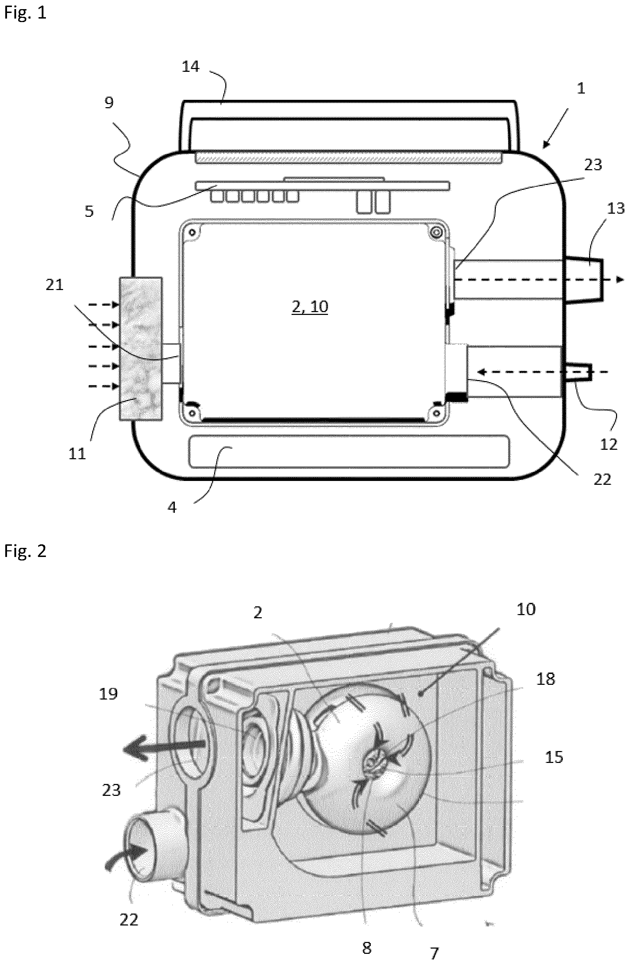

Fig. 1 schématise un mode de réalisation d'un appareil d'assistance respiratoire 1, aussi appelé ventilateur médical 1, équipé d'une micro-soufflante 2 motorisée 2 (visible sur Fig. 2), encore appelée soufflante, turbine ou compresseur, d'un système de contrôle 5 selon l'invention, et d'une alimentation électrique 4, par exemple une (ou plusieurs) batterie(s) et/ou des moyens de raccordement au secteur (110/220 V), tels que cordon électrique, un convertisseur AC/DC et une prise de raccordement au secteur ou autres. De préférence, l'alimentation électrique est de type AC/DC fournissant une tension de l'ordre de 28 V.

Par exemple, le ventilateur médical 1 peut être un appareil d'assistance respiratoire délivrant du gaz à un ou plusieurs niveaux de pression, c'est-à-dire de type CPAP ou BPAP. De façon générale, le ventilateur médical 1 est destiné à traiter des pathologies respiratoires chez des patients humains, adultes et/ou enfants.

La micro-soufflante 2 motorisée est, dans ce mode de réalisation, agencée dans un caisson interne 10 lui-même inséré dans la carcasse périphérique 9 ou coque externe du ventilateur médical 1. Selon un autre mode de réalisation, la micro-soufflante 2 peut être installée directement dans la carcasse 9 du ventilateur médical 1, c'est-à-dire sans utiliser de caisson 10.

Par ailleurs, la carcasse 9 du ventilateur médical 1 comprend, dans ce mode de réalisation, une entrée d'air 11 servant à l'alimentation en air de la micro-soufflante 2, une entrée d'oxygène 12 servant à fournir de l'oxygène afin de pouvoir enrichir l'air en oxygène et obtenir ainsi des mélanges air/O₂, et une sortie de gaz respiratoire 13 permettant d'alimenter le patient en gaz respiratoire, typiquement de l'air ou un mélange air/O₂, provenant de la sortie de la micro-soufflante 2.

L'entrée d'oxygène 12 du ventilateur médical 1 est en communication fluidique avec une entrée d'oxygène 22 du caisson 10 et, de manière analogue, l'entrée d'air 11 et la sortie de gaz respiratoire 13 du ventilateur 1 sont en communication fluidique avec, respectivement, une entrée d'air 21 et une sortie de gaz respiratoire 23 du caisson 10. Selon un autre mode de réalisation, on peut prévoir que l'injection d'oxygène ait lieu plutôt en aval de la micro-soufflante.

Optionnellement, la carcasse 9 du ventilateur médical 1 peut comprendre une poignée 14 de portage facilitant son transport par un utilisateur.

Bien entendu, le ventilateur médical 1 peut aussi comprendre d'autres éléments ou composants classiques (non schématisés) utilisés couramment dans ce type d'appareils, tel que des capteurs de pression et/ou débit, des organes de commande pneumatique, un écran de visualisation, une interface homme-machine (IHM) permettant de régler ou sélectionner des paramètres ventilatoires (niveaux de pression, volumes, modes ventilatoires...) comprenant des organes de sélection (e.g. boutons, touches digitales, curseurs...), un bouton de mise en marche ou d'arrêt (i.e. bouton « on/off ») du ventilateur 1, etc...

Fig. 2 illustre l'agencement de la micro-soufflante 2 motorisée au sein du caisson 10. Le caisson 10 est formé ici de deux parties de caisson, c'est-à-dire deux demi-caissons venant se fixer l'un à l'autre, par exemple en prenant la micro-soufflante 1 motorisée en 'sandwich'. Bien entendu, une architecture différente du caisson est possible. Le caisson 10 peut comprendre d'autres éléments, notamment un ou des circuits de gaz ou analogues, des capteurs ou autres. Le caisson 10 est optionnel, c'est-à-dire que la micro-soufflante 2 pourrait être agencée directement dans la carcasse 10 du ventilateur 1.

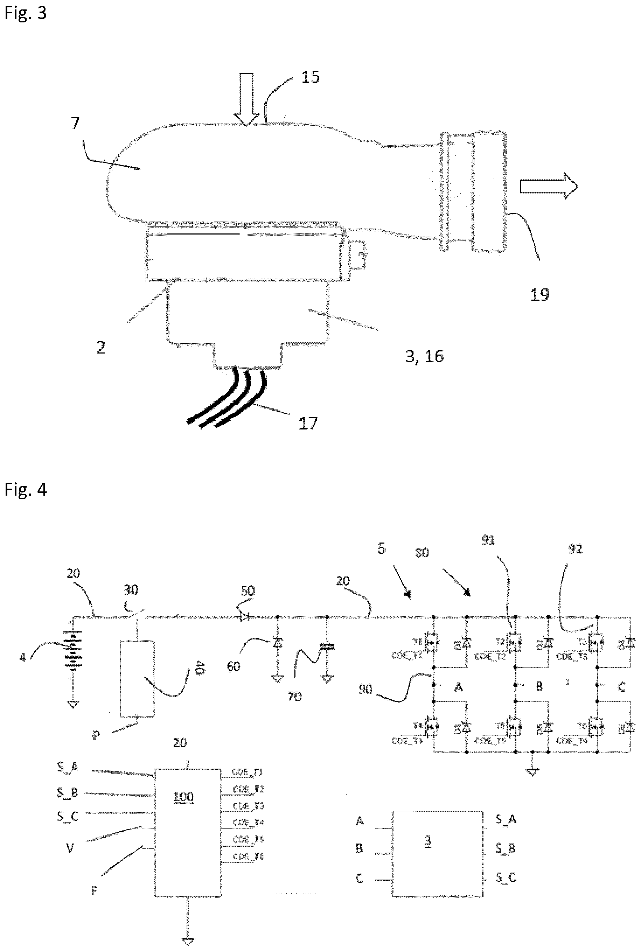

Comme schématisé en Fig. 3, la micro-soufflante 2 motorisée comprend un carter 16 renfermant un moteur électrique (non visible) et servant à le protéger, en particulier le stator et le rotor du moteur. Le carter 16 externe peut être formée d'un alliage métallique, par exemple un alliage d'aluminium ou du ZAMAK (alliage zinc, aluminium, magnésium et cuivre), ou de polymère ou de thermoplastique, par exemple du PET ou PA. Le carter 16 a généralement une section circulaire, c'est-à-dire une forme générale cylindrique. Il peut aussi comporter des ailettes ou structures de refroidissement faisant office de radiateur.

Le moteur électrique 3 est un moteur rotatif, typiquement à rotor et stator, préférentiellement sans balai ('brushless' en anglais). Il est conçu pour atteindre une vitesse de rotation, en fin d'accélération, de l'ordre de 30 000 à 40 000 tr/min, voire jusqu'à 50 000 à 70 000 tr/min. Le contrôle du fonctionnement (i.e. accélérations et décélérations/freinage) moteur électrique 3 est expliqué ci-après.

L'alimentation en courant électrique du moteur électrique 3 de la micro-soufflante 2 pour permettre son fonctionnement, se fait de manière classique au moyen d'une connectique adaptée 17 incluant un bus d'alimentation 20, par exemple des fils ou câbles électriques, amenant le courant électrique depuis l'alimentation électrique 4 (cf. Fig. 1), par exemple une (ou plusieurs) batterie(s) d'alimentation électrique, comme expliqué ci-avant, jusqu'au moteur électrique 3.

Pendant son fonctionnement, le moteur de la micro-soufflante 1 entraîne en rotation, un arbre ou axe rotatif, aussi appelé 'axe-moteur', portant une roue à ailettes, aussi appelée 'roue à pales'. Cette roue à ailettes a de préférence une section circulaire de diamètre compris entre 20 et 80 mm, typiquement entre 30 et 60 mm. La roue à ailettes comprend une première face portant des ailettes, aussi appelées 'pales' et une deuxième face dépourvue d'ailettes, c'est-à-dire plane, qui est en outre situé en regard du moteur et du carter 16. La roue à ailettes est agencée de manière à être mobile en rotation au sein du compartiment interne 18 de la volute 7 surmontant le moteur électrique 3 et le carter 16, de manière à générer un flux gazeux à une pression supérieure à la pression atmosphérique (> 1 atm).

Préférentiellement, la roue à ailettes et la volute 7 sont en matériau(x) polymère, par exemple un thermoplastique (e.g. PEEK, PA, ...), ou en alliage métallique, par exemple un alliage à base aluminium.

Selon le mode de réalisation choisi, la volute 7 peut être formée de deux demi-volutes, à savoir une demi-volute inférieure et une demi-volute supérieure assemblées et fixées de manière solidaire et étanche l'une à l'autre, par exemple par collage ou autre. Un joint d'étanchéité peut être interposé entre ces demi-volutes. La volute 7 a une section générale circulaire. Elle comprend en outre une ouverture supérieure 15 par lequel le gaz, typiquement de l'air ou un mélange air/O₂, est aspiré dans le compartiment interne 18 de la volute 7, lors des rotations de la roue, et un conduit de sortie 19 par lequel le flux de gaz respiratoire généré dans le compartiment 18, lors des rotations de la roue, ressort de la micro-soufflante 2. Le mélange air/O₂ entrant dans la volute 7 est préférentiellement réalisé en amont de l'ouverture supérieure 15, par exemple dans une chambre de mélange (non montrée) aménagée dans le caisson 10.

Le conduit de sortie 19 est en communication fluidique avec la sortie de gaz respiratoire 23 du caisson 10 et la sortie de gaz respiratoire 13 du ventilateur 1 de sorte que le gaz sortant du compartiment interne 18 de la volute 7 circule successivement dans le conduit de sortie 19, la sortie de gaz respiratoire 23 du caisson 10 et la sortie de gaz respiratoire 13 du ventilateur 1, avant d'être acheminé jusqu'aux voies aériennes du patient via un tuyau flexible ou analogue alimentant une interface respiratoire, tel un masque respiratoire ou une sonde trachéale par exemple.

Pendant le fonctionnement du ventilateur médical 1, le moteur électrique 3 de la micro-soufflante 2 est piloté par le système de contrôle 5 du ventilateur médical 1 faisant office de moyens de pilotage.

Comme détaillé ci-après, le système de contrôle 5 comprend une (ou des) carte électronique à microprocesseur(s), typiquement à microcontrôleur, mettant en oeuvre un ou des algorithmes. Le système de contrôle 5 est configuré pour commander le fonctionnement du moteur électrique 3. Il est alimenté en courant électrique provenant de l'alimentation électrique 4.

Plus précisément, le moteur électrique 3 de la micro-soufflante 2 est commandé par le système de contrôle 5 pour opérer cycliquement des phases d'accélération et de décélération/freinage successives de manière à se calquer sur les phases inspiratoires et expiratoires du patient.

Or, lors des phases de freinage, c'est-à-dire des décélérations/ralentissements, du moteur électrique 3, des surtensions électriques ou pics d'énergie importantes sont générées par le moteur 3, lors du relâchement du court-circuit de chaque phase moteur en situation de freinage. De l'énergie emmagasinée dans les bobines du moteur électrique 3 est alors subitement relâchée dans le bus d'alimentation 20 reliant la source de courant 4 au moteur 3 électrique de la micro-soufflante 2, en provoquant alors une élévation de la tension d'alimentation générale et un échauffement conséquent des composants situés sur ce bus 20, ce qui peut les détériorer.

Pour y remédier, selon l'invention, comme illustré en Fig. 4, le système de contrôle 5 comprend un circuit à ponts de transistors 80, agencé entre l'alimentation électrique 4 et le moteur électrique 3 de la micro-soufflante 2.

Le circuit à ponts de transistors 80 comprenant 3 ponts de transistors 90, 91, 92 qui sont agencés en parallèle les uns des autres. Chaque pont de transistors 90, 91, 92 relient le bus 20 au moteur 3 de la micro-soufflante 2, et comprend un couple de transistors T1/T4, T2/T5, T3/T6, c'est-à-dire trois couples de transistors T1-T6. Les ponts de transistors 90, 91, 92 servent à piloter la rotation du moteur 3 de la micro-soufflante 2.

Plus précisément, le circuit à ponts de transistors 80 comprend un premier, un deuxième et un troisième pont de transistors 90, 91, 92 qui sont respectivement reliés aux première, deuxième et troisième phases A, B, C du moteur 3, comme illustré en Fig. 4. Les première, deuxième et troisième phases A, B, C du moteur 3 sont, respectivement, reliées électriquement aux premier, deuxième et troisième ponts de transistors 90, 91, 92, respectivement entre les deux transistors T1-T6 de chaque couple de transistors T1-T6, et par ailleurs, entre les deux diodes de régulation de tension D1-D6 des couples diodes de régulation de tension D1-D6 agencés en parallèle desdits transistors T1-T6.

Les diodes de régulation de tension D1-D6, à savoir ici 6 diodes, sont agencées en parallèle des transistors T1-T6 pour limiter au moins une partie des surtensions ou anlogue du moteur 3, lors des freinages/décélérations du moteur 3 de la micro-soufflante 2. Préférentiellement, les diodes de régulation de tension D1-D6 sont des diodes Zener.

D'une façon générale, une diode Zener est un assemblage de semiconducteurs autorisant le passage du courant dans le sens direct, i.e. sens normal, mais aussi dans le sens inverse lorsque la tension à ses bornes est plus élevée que son seuil de tension inverse, i.e. tension Zener ou d'avalanche, à partir duquel se déclenche l'effet d'avalanche de la diode, par exemple un seuil de l'ordre de 30 à 33 V dans le cadre de la présente invention. Contrairement à une diode TVS, une diode Zener n'absorbe pas le courant transitoire mais maintient ou limite la tension au niveau de son seuil. A partir du moment où la tension devient supérieure à sa tension de maintien, elle va absorber tout le courant pour limiter cette tension offrant ainsi une tension de régulation relativement fixe. Une diode Zener est donc une diode de régulation de tension.

Comme illustré en Fig. 4, il prévu également un circuit de pilotage 100 du moteur pour commander au moins les accélérations et les décélérations du moteur 3 de la micro-soufflante 2, lequel est relié électriquement au moteur 3, via les liaisons S_A, S_B et S_C, et aux 6 transistors T1-T6, via les liaisons CDE_T1/T4, CDE_T2/T5, et CDE_T3/T6.

Le circuit de pilotage 100 du moteur comprend au moins un microprocesseur, de préférence au moins un microcontrôleur, qui met en oeuvre un ou plusieurs algorithmes. Il est agencé sur au moins une carte électronique.

Le circuit de pilotage 100 du moteur, en particulier le microcontrôleur, pilote aussi la vitesse du moteur 3 de la micro-soufflante 2, c'est-à-dire le régime stabilisé du moteur 3. Pour ce faire, le circuit de pilotage 100, en particulier le microcontrôleur génère une modulation de largeur d'impulsion (*PWM* en anglais pour Pulse *Width Modulation*) de tension moyenne entre 0 et 5 V environ qui commande la vitesse du moteur 3 de la micro-soufflante 2.

Les moyens de pilotage 100 sont alimentés en courant électrique par le bus 20, via un dispositif abaisseur de tension agencé entre eux.

Le circuit de pilotage du moteur 100 comprend en outre des entrées et des sorties, à savoir :
- une sortie de commande de freinage F permettant d'activer le freinage en commandant seulement les transistors T4, T5 et T6 du circuit à ponts de transistors 80.
- 3 entrées S_A, S_B et S_C connectées aux capteurs à effet hall présents dans le moteur 3 de la micro-soufflante 2 et permettent d'indiquer la position du rotor du moteur 3 et, avec la consigne de vitesse, de positionner les niveaux (par exemple 0 ou 1) sur les commandes (CDE_T1, CDE_T2, CDE_T3, CDE_T4, CDE_T5 et CDE_T6) des transistors T1-T6.
- une entrée V de commande de vitesse connectée à la sortie commande de vitesse du microcontrôleur du circuit de pilotage 100, laquelle fixe la vitesse du moteur 3 de la micro-soufflante 2 en fonction du cycle de ventilation du patient.

Le circuit de pilotage 100 du moteur peut comprendre une ou plusieurs cartes électroniques dont une carte portant le circuit à ponts de transistors 80.

Agencés entre l'alimentation électrique 4 et le moteur électrique 3, sont également prévus un dispositif interrupteur 30, par exemple un transistor MOS, qui est configuré pour contrôler le passage du courant dans le bus d'alimentation 20 en direction du moteur 3 et un circuit limiteur de courant 40 qui est quant à lui configuré pour limiter le courant circulant dans le bus 20 en direction du moteur 3, lors de la fermeture du dispositif interrupteur 30. L'alimentation électrique 4 est couplée au dispositif interrupteur 30.

Le circuit limiteur de courant 40 comprend un circuit RC à résistance et condensateur, qui permet de fixer la constante de temps de commande du dispositif interrupteur 30. Le circuit limiteur de courant 40 est lui-même commandé par le microcontrôleur du circuit de pilotage 100, via l'entrée P du circuit limiteur de courant 40.

De plus, le système de contrôle 5 comprend aussi, agencées entre l'alimentation électrique 4 et le moteur électrique 3, une diode de blocage de courant remontant 50, par exemple une diode Schottky ou analogue ayant un seuil de tension direct assez bas, par exemple moins de 0,7 V, configurée pour bloquer le courant généré par le moteur 3 et circulant dans le bus d'alimentation 20 en direction de l'alimentation électrique 4, lors des décélérations du moteur 3, et une diode d'écrêtage 60 ou diode de régulation de tension additionnelle, par exemple une diode Zener ou analogue, configurée pour limiter toute surtension éventuelle de l'alimentation électrique 4. Le seuil de la diode d'écrêtage 60 est préférentiellement de l'ordre de 30 à 33 V.

Alternativement, en lieu et place de la diode de blocage de courant remontant 50, on peut utiliser un transistor MOSFET et un driver ou contrôleur de diode idéale.

Enfin, un (ou des) condensateur(s) 70 est agencé entre l'alimentation électrique 4 et le moteur électrique 3. Il permet de fournir du courant électrique au moteur 3 électrique rotatif de la micro-soufflante 2, lors des appels de courant au moment des accélérations du moteur 3, et pour limiter au moins une partie des surtensions du moteur 3 lors des décélérations dudit moteur 3 de la micro-soufflante 2.

Comme on le voit sur la Fig. 4, le dispositif interrupteur 30 et la diode de blocage de courant remontant 50 sont agencés sur le bus d'alimentation 20 sur la branche (+) du bus.

Une telle architecture du système de contrôle de la micro-soufflante motorisée d'un appareil d'assistance respiratoire ou ventilateur médical selon l'invention permette un contrôle efficace des accélérations et décélérations/freinages de la micro-soufflante tout en étant protégé efficacement contre les surtensions électriques, les pics d'énergie ou analogues, qui sont engendrés lors des phases de décélération/freinage du moteur électrique de la micro-soufflante, ce qui permet d'éviter ou de limiter la détérioration des composants électroniques sensibles à ces surtensions électriques ou similaires.

## Revendications

1. Appareil d'assistance respiratoire (1) comprenant :
- une micro-soufflante (2) équipée d'un moteur électrique (3),
- un système de contrôle de moteur (5) pour contrôler le moteur électrique (3) de la micro-soufflante (2), et
- une alimentation électrique (4) alimentant électriquement le moteur électrique (3) et le système de contrôle (5) via un bus d'alimentation (20) reliant l'alimentation électrique (4) au moteur électrique (3) de la micro-soufflante (2),
**caractérisé en ce que** le système de contrôle (5) comprend :
- un circuit à ponts de transistors (80), agencé entre l'alimentation électrique (4) et le moteur électrique (3), comprenant 3 ponts de transistors (90, 91, 92) agencés en parallèle, chaque pont de transistors (90, 91, 92) reliant le bus (20) au moteur (3) et comprenant un couple de transistors (T1, T4 ; T2, T5 ; T3, T6),
- un circuit de pilotage du moteur (100) pour commander au moins les accélérations et les décélérations du moteur (3), ledit circuit de pilotage du moteur (100) étant relié électriquement au moteur (3) et aux transistors (T1, T4 ; T2, T5 ; T3, T6), et
- une pluralité de diodes de régulation de tension (D1-D6) agencées en parallèle des transistors (T1, T4 ; T2, T5 ; T3, T6) pour limiter au moins une partie des surtensions du moteur (3) lors des décélérations du moteur (3).

2. Appareil selon la revendication 1, **caractérisé en ce qu'**il comprend en outre, agencés entre l'alimentation électrique (4) et le moteur électrique (3), un dispositif interrupteur (30) pour contrôler le passage du courant dans le bus d'alimentation (20) en direction du moteur (3).

3. Appareil selon l'une des revendications précédentes, **caractérisé en ce qu'**il comprend en outre agencées entre l'alimentation électrique (4) et le moteur électrique (3) au moins une diode de blocage de courant remontant (50) pour bloquer le courant généré par le moteur (3) et circulant dans le bus d'alimentation (20) en direction de l'alimentation électrique (4), lors des décélérations du moteur (3).

4. Appareil selon l'une des revendications précédentes, **caractérisé en ce qu'**il comprend en outre agencées entre l'alimentation électrique (4) et le moteur électrique (3), au moins une diode d'écrêtage (60) pour limiter au moins une partie des surtensions (20) de l'alimentation électrique (4) circulant dans le bus (20).

5. Appareil selon l'une des revendications précédentes, **caractérisé en ce qu'**il comprend en outre au moins un condensateur (70), agencée entre l'alimentation électrique (4) et le moteur électrique (3), pour fournir du courant au moteur (3), lors des accélérations du moteur (3), et pour limiter au moins une partie des surtensions du moteur (3) lors des décélérations du moteur (3).

6. Appareil selon l'une des revendications précédentes, **caractérisé en ce que** le dispositif interrupteur (30) et/ou la ou les diodes de blocage de courant remontant (50) sont agencés sur le bus d'alimentation (20), en particulier sur la branche (+) du bus.

7. Appareil selon l'une des revendications précédentes, **caractérisé en ce qu'**il comprend en outre un circuit limiteur de courant (40) pour limiter le courant circulant dans le bus (20) vers le moteur (3), lors de la fermeture du dispositif interrupteur (30).

8. Appareil selon l'une des revendications précédentes, **caractérisé en ce qu'**au moins une diode de blocage de courant remontant (50) est au moins une diode Schottky.

9. Appareil selon l'une des revendications précédentes, **caractérisé en ce qu'**au moins une diode d'écrêtage (60) est au moins une diode Zener.

10. Appareil selon l'une des revendications précédentes, **caractérisé en ce que** le circuit à ponts de transistors (80) comprend un premier, un deuxième et un troisième ponts de transistors (90, 91, 92) et le moteur (3) comprend une première, une deuxième et une troisième phases (A, B, C), les premier, deuxième et troisième ponts de transistors (90, 91, 92) étant respectivement reliés aux première, deuxième et troisième phases (A, B, C) du moteur (3).

11. Appareil selon la revendication 10, **caractérisé en ce que** les première, deuxième et troisième phases (A, B, C) du moteur (3) sont, respectivement, reliées électriquement aux premier, deuxième et troisième ponts de transistors (90, 91, 92), respectivement :
- entre les deux transistors (T1-T6) de chaque couple de transistors (T1, T4 ; T2, T5 ; T3,T6) et
- entre les deux diodes de régulation de tension (D1-D6) des couples diodes de régulation de tension (D1, D4 ; D2, D5 ; D3, D6) agencés en parallèle desdits transistors (T1, T4 ; T2, T5 ; T3,T6).

12. Appareil selon l'une des revendications précédentes, **caractérisé en ce que** les diodes de régulation de tension (D1-D6) sont des diodes Zener.

13. Appareil selon l'une des revendications précédentes, **caractérisé en ce que** la micro-soufflante (2) est équipée d'un moteur électrique sans balai.

14. Appareil selon l'une des revendications précédentes, **caractérisé en ce que** le circuit de pilotage du moteur (100) comprend un microprocesseur, de préférence un microcontrôleur, mettant en oeuvre un ou plusieurs algorithmes.

15. Installation de fourniture de gaz respiratoire à un patient comprenant un appareil d'assistance respiratoire (1) selon l'une des revendications précédentes, un conduit flexible raccordé fluidiquement audit appareil d'assistance respiratoire (1) et alimenté en gaz par la micro-soufflante (2) dudit appareil d'assistance respiratoire (1), et une interface respiratoire () alimentée en gaz par ledit conduit flexible.

## Patentansprüche

1. Atmungsunterstützungsgerät (1), umfassend:
- ein Mikrogebläse (2), das mit einem Elektromotor (3) ausgestattet ist,
- ein Motorsteuerungssystem (5) zum Steuern des Elektromotors (3) des Mikrogebläses (2) und
- eine Stromversorgung (4), die den Elektromotor (3) und das Steuerungssystem (5) über einen Versorgungsbus (20), der die Stromversorgung (4) mit dem Elektromotor (4) des Mikrogebläses (2) verbindet, elektrisch versorgt, **dadurch gekennzeichnet, dass** das Steuerungssystem (4) umfasst:
- eine Transistorbrückenschaltung (80), die zwischen der Stromversorgung (4) und dem Elektromotor (3) angeordnet ist und 3 parallel angeordnete Transistorbrücken (90, 91, 92) umfasst, wobei jede Transistorbrücke (90, 91, 92) den Bus (20) mit dem Motor (3) verbindet und ein Transistorpaar (T1, T4; T2, T5; T3, T6) umfasst,
- eine Ansteuerschaltung des Motors (100), um mindestens die Beschleunigungen und die Verzögerungen des Motors (3) zu steuern, wobei die Ansteuerschaltung des Motors (100) mit dem Motor (3) und mit den Transistoren (T1, T4; T2, T5; T3, T6) elektrisch verbunden ist, und
- eine Mehrzahl von Spannungsregelungsdioden (D1-D6), die parallel zu den Transistoren (T1, T4; T2, T5; T3, T6) angeordnet sind, um mindestens einen Teil der Überspannungen des Motors (3) bei Verzögerungen des Motors (3) zu begrenzen.

2. Gerät nach Anspruch 1, **dadurch gekennzeichnet, dass** es ferner, angeordnet zwischen der Stromversorgung (4) und dem Elektromotor (3), eine Unterbrechervorrichtung (30) umfasst, um den Durchgang des Stroms im Versorgungsbus (20) in Richtung des Motors (3) zu steuern.

3. Gerät nach einem der vorhergehenden Ansprüche, **dadurch gekennzeichnet, dass** es ferner, angeordnet zwischen der Stromversorgung (4) und dem Elektromotor (3), mindestens eine Rückstromsperrdiode (50) umfasst, um den von dem Motor (3) erzeugten und im Versorgungsbus (20) in Richtung der Stromversorgung (4) fließenden Strom bei Verzögerungen des Motors (3) zu sperren.

4. Gerät nach einem der vorhergehenden Ansprüche, **dadurch gekennzeichnet, dass** es ferner, angeordnet zwischen der Stromversorgung (4) und dem Elektromotor (3) mindestens eine Begrenzungsdiode (60) umfasst, um mindestens einen Teil der Überspannungen (20) der Stromversorgung (4), die in dem Bus (20) fließen, zu begrenzen.

5. Gerät nach einem der vorhergehenden Ansprüche, **dadurch gekennzeichnet, dass** es ferner mindestens einen Kondensator (70) umfasst, der zwischen der Stromversorgung (4) und dem Elektromotor (3) angeordnet ist, um an den Motor (3) bei Beschleunigungen des Motors (3) Strom abzugeben und um mindestens einen Teil der Überspannungen des Motors (3) bei Verzögerungen des Motors (3) zu begrenzen.

6. Gerät nach einem der vorhergehenden Ansprüche, **dadurch gekennzeichnet, dass** die Unterbrechervorrichtung (30) und/oder die Rückstromsperrdiode(n) (50) auf dem Versorgungsbus (20) angeordnet sind, insbesondere auf dem Zweig (+) des Busses.

7. Gerät nach einem der vorhergehenden Ansprüche, **dadurch gekennzeichnet, dass** es ferner eine Strombegrenzerschaltung (40) umfasst, um den im Bus (20) zu dem Motor (3) hin fließenden Strom beim Schließen der Unterbrechervorrichtung (30) zu begrenzen.

8. Gerät nach einem der vorhergehenden Ansprüche, **dadurch gekennzeichnet, dass** mindestens eine Rückstromsperrdiode (50) mindestens eine Schottky-Diode ist.

9. Gerät nach einem der vorhergehenden Ansprüche, **dadurch gekennzeichnet, dass** mindestens eine Begrenzungsdiode (60) mindestens eine Zener-Diode ist.

10. Gerät nach einem der vorhergehenden Ansprüche, **dadurch gekennzeichnet, dass** die Transistorbrückenschaltung (80) eine erste, eine zweite und eine dritte Transistorbrücke (90, 91, 92) umfasst und der Motor (3) eine erste, eine zweite und eine dritte Phase (A, B, C) umfasst, wobei die erste, die zweite und die dritte Transistorbrücke (90, 91, 92) mit der ersten, der zweiten beziehungsweise der dritten Phase (A, B, C) des Motors (3) verbunden sind.

11. Gerät nach Anspruch 10, **dadurch gekennzeichnet, dass** die erste, die zweite und die dritte Phase (A, B, C) des Motors (3) jeweils elektrisch mit der ersten, der zweiten beziehungsweise der dritten Transistorbrücke (90, 91, 92) verbunden sind:
- zwischen den beiden Transistoren (T1-T6) jedes Transistorpaars (T1, T4; T2, T5; T3, T6) und
- zwischen den beiden Spannungsregelungsdioden (D1-D6) der Spannungsregelungsdiodenpaare (D1, D4; D2, D5; D3, D6), die parallel zu den Transistoren (T1, T4; T2, T5; T3, T6) angeordnet sind.

12. Gerät nach einem der vorhergehenden Ansprüche, **dadurch gekennzeichnet, dass** die Spannungsregelungsdioden (D1-D6) Zener-Dioden sind.

13. Gerät nach einem der vorhergehenden Ansprüche, **dadurch gekennzeichnet, dass** das Mikrogebläse (2) mit einem bürstenlosen Elektromotor ausgestattet ist.

14. Gerät nach einem der vorhergehenden Ansprüche, **dadurch gekennzeichnet, dass** die Ansteuerschaltung des Motors (100) einen Mikroprozessor, bevorzugt einen Mikrocontroller, umfasst, der ein oder mehrere Algorithmen ausführt.

15. Anlage zur Abgabe von Atemgas an einen Patienten, umfassend ein Atmungsunterstützungsgerät (1) nach einem der vorhergehenden Ansprüche, einen Schlauch, der fluidisch an das Atmungsunterstützungsgerät (1) angeschlossen ist und durch das Mikrogebläse (2) des Atmungsunterstützungsgeräts (1) mit Gas versorgt wird, und eine Atemschnittstelle (), die durch den Schlauch mit Gas versorgt wird.

## Claims

1. Device (1) for assisting breathing, comprising:
- a micro-blower (2) equipped with an electric motor (3),
- a motor control system (5) for controlling the electric motor (3) of the micro-blower (2), and
- a power supply (4) that electrically powers the electric motor (3) and control system (5) via a supply bus (20) connecting the power supply (4) to the electric motor (3) of the micro-blower (2),
**characterized in that** the control system (5) comprises:
- a transistor bridge circuit (80), arranged between the power supply (4) and the electric motor (3), comprising 3 transistor bridges (90, 91, 92) arranged in parallel, each transistor bridge (90, 91, 92) connecting the bus (20) to the motor (3) and comprising one pair of transistors (T1, T4; T2, T5; T3, T6),
- a motor drive circuit (100) for commanding at least the accelerations and decelerations of the motor (3), said motor drive circuit (100) being electrically connected to the motor (3) and to the transistors (T1, T4; T2, T5; T3, T6), and
- a plurality of voltage-regulating diodes (D1-D6), arranged in parallel with the transistors (T1, T4; T2, T5; T3, T6), for limiting at least some of the overvoltages of the motor (3) during decelerations of the motor (3).

2. Device according to Claim 1, **characterized in that** it further comprises, arranged between the power supply (4) and the electric motor (3), a switching device (30) for controlling passage of current through the supply bus (20) towards the motor (3).

3. Device according to one of the preceding claims, **characterized in that** it further comprises, arranged between the power supply (4) and the electric motor (3), at least one rising-current blocking diode (50) for blocking the current generated by the motor (3) and flowing through the power supply bus (20) towards the power supply (4), during decelerations of the motor (3).

4. Device according to one of the preceding claims, **characterized in that** it further comprises, arranged between the power supply (4) and the electric motor (3), at least one clipping diode (60) for limiting at least some of the overvoltages (20) of the power supply (4) flowing through the bus (20).

5. Device according to one of the preceding claims, **characterized in that** it further comprises at least one capacitor (70), arranged between the power supply (4) and the electric motor (3), for delivering current to the motor (3), during accelerations of the motor (3), and for limiting at least some of the overvoltages of the motor (3), during decelerations of the motor (3).

6. Device according to one of the preceding claims, **characterized in that** the switching device (30) and/or the one or more rising-current blocking diodes (50) are arranged on the supply bus (20), and in particular on the (+) branch of the bus.

7. Device according to one of the preceding claims, **characterized in that** it further comprises a current-limiting circuit (40) for limiting the current flowing through the bus (20) towards the motor (3), during closure of the switching device (30).

8. Device according to one of the preceding claims, **characterized in that** at least one rising-current blocking diode (50) is at least one Schottky diode.

9. Device according to one of the preceding claims, **characterized in that** at least one clipping diode (60) is at least one Zener diode.

10. Device according to one of the preceding claims, **characterized in that** the transistor bridge circuit (80) comprises a first, a second and a third transistor bridge (90, 91, 92) and the motor (3) comprises a first, a second and a third phase (A, B, C), the first, second and third transistor bridges (90, 91, 92) being connected to the first, second and third phases (A, B, C) of the motor (3), respectively.

11. Device according to Claim 10, **characterized in that** the first, second and third phases (A, B, C) of the motor (3) are electrically connected to the first, second and third transistor bridges (90, 91, 92), respectively:
- between the two transistors (T1-T6) of each pair of transistors (T1, T4; T2, T5; T3, T6) and
- between the two voltage-regulating diodes (D1-D6) of the pairs of voltage-regulating diodes (D1, D4; D2, D5; D3, D6) arranged in parallel with said transistors (T1, T4; T2, T5; T3, T6) .

12. Device according to one of the preceding claims, **characterized in that** the voltage-regulating diodes (D1-D6) are Zener diodes.

13. Device according to one of the preceding claims, **characterized in that** the micro-blower (2) is equipped with a brushless electric motor.

14. Device according to one of the preceding claims, **characterized in that** the motor drive circuit (100) comprises a microprocessor, and preferably a microcontroller, implementing one or more algorithms.

15. Installation for delivering breathing gas to a patient, comprising a device (1) for assisting breathing according to one of the preceding claims, a flexible duct fluidly connected to said device (1) for assisting breathing and supplied with gas by the micro-blower (2) of said device (1) for assisting breathing, and a respiratory interface () supplied with gas via said flexible duct.
